# EUROPÄISCHE PATENTSCHRIFT

(11) **EP 0 880 916 B1**
(45) Veröffentlichungstag und Bekanntmachung des Hinweises auf die Patenterteilung: **27.06.2001**
(21) Anmeldenummer: 98108407.2
(22) Anmeldetag: 08.05.1998
(51) Int. Cl.: A45D 7/04

(54) **Haardauerverformungsverfahren mit zunächst höherem und später niedrigerem pH-Wert in der Reduktionsstufe**
Process for permanent waving of hair
Procédé pour l'ondulation permanent des cheveux

(30) Priorität: 31.05.1997 DE 19722860; 31.05.1997 DE 19722861
(43) Veröffentlichungstag der Anmeldung: 02.12.1998
(73) Patentinhaber: Wella Aktiengesellschaft, 64274 Darmstadt (DE)
(72) Erfinder: Buheitel, Horst, 95111 Rehau (DE); Mager, Herbert, Dr., 1723 Marly (CH); Lang, Günther, Dr., 64354 Reinheim (DE); Galley, Jean-Luc, 1752 Villars-sur-Glane (CH)

(56) Entgegenhaltungen:
- EP-A- 0 443 356
- US-A- 4 982 749

## Beschreibung

Die vorliegende Erfindung betrifft ein Verfahren zum schonenden dauerhaften Verformen von menschlichen Haaren unter Anwendung einer Zwischenspülung, bei dem man das Haar zunächst für eine kurze Zeit im alkalischen Bereich und sodann für eine längere Zeit im sauren Bereich reduziert, mit verbesserten Pflege- und Welleigenschaften.

Der Behandlungsablauf bei Verfahren zur dauerhaften Umformung von menschlichem Haar ist üblicherweise wie folgt: Die Haare werden zuerst gewaschen und dann handtuchgetrocknet. Anschließend wird das Haar im noch etwas feuchten Zustand abgeteilt und partienweise auf zylindrische Wickler aufgerollt. Danach wird auf die Wickel eine Lösung mit einer oder mehreren keratinreduzierenden Substanzen aufgetragen, etwa 5 bis 40 Minuten lang bei einer Temperatur zwischen 15 und 50 Grad Celsius einwirken gelassen, und danach wieder ausgespült. Als alternative Anwendung hierzu kann die reduzierende Lösung schon vor dem Aufwickeln auf die Haare gebracht werden. In diesem Fall spricht man von Vorfeuchten. Nach der Einwirkung der reduzierenden Lösung wird diese mit warmem Wasser ausgespült, anschließend das eingewickelte Haar mit einem Handtuch leicht angetrocknet und danach mit einer wäßrigen Lösung eines Oxidationsmittels 1 bis 15 Minuten lang, bevorzugt bei Raumtemperatur, behandelt. Danach wird das Oxidationsmittel ebenfalls ausgespült und die Wickler entfernt. Falls notwendig, kann jetzt die Behandlung mit dem Oxidationsmittel wiederholt werden. Nach der Einwirkung und dem Ausspülen des Oxidationsmittels haben die Haare eine bleibende, gewellte Form angenommen.

Dabei erfolgt Umformung in zwei Stufen: Die reduktive Spaltung der Cystin-Disulfidbrücken des Haares durch Einwirkung des Reduktionsmittels und die anschließende Fixierung beziehungsweise Neutralisierung durch Aufbringung des Oxidationsmittels, wodurch die Cystin-Disulfidbrücken wiederhergestellt werden.

Das klassische Reduktionsmittel ist dabei die Thioglykolsäure, entweder als freie Säure oder in Form ihrer Salze, insbesondere des Ammoniumsalzes, wobei der pH-Wert dieser Zusammensetzung üblicherweise im alkalischen Bereich zwischen pH 8 und pH 10 liegt. Diese thioglykolathaltigen Zusammensetzungen können bei wiederholten, zeitlich nahe zusammenliegenden Anwendungen zu einer erhöhten Haarschädigung führen. Als weitere haarkeratinreduzierende Wirkstoffe können auch Sulfite, Thiomilchsäure, 3-Mercaptopropionsäure, Mercaptocarbonsäureester oder Cystein verwendet werden.

Die Fixierung beziehungsweise Neutralisierung des durch die Einwirkung von Thioverbindungen verformten Haares wird durch Einwirkung von Oxidationsmitteln, insbesondere Wasserstoffperoxid, vorgenommen.

Obwohl sich die genannten Haarverformungsverfahren weiter Verbreitung erfreuen, besteht immer noch ein Bedürfnis, die Qualität der Verformung hinsichtlich Elastizität und Sprungkraft der Locke und der Pflegeeigenschaften, wie Griff und Kämmbarkeit des Haares nach Anwendung solcher Verfahren, zu verbessern.

Bei jeder Wellbehandlung ist es neben der gewünschten Umformung ein wichtiges Ziel, die Haare möglichst wenig zu schädigen. Um dies zu erreichen werden als keratinreduzierende Substanzen unter anderem Thioglykolsäuremonoglycerinester und/oder Cysteamin eingesetzt. Diese Substanzen haben die Eigenschaft, bei relativ niedrigen pH-Werten von 6,8 bis 8,0 eine ausreichende Haarumformung bei vergleichsweise geringer Haarschädigung zu ermöglichen. Dies ist ein wesentlicher Vorteil, dem allerdings gravierende Nachteile gegenüberstehen. So ist der Glycerinester der Thioglykolsäure sensibilisierend, während die Anwendung von Cysteamin zu einem bleibenden, sehr unangenehmen Geruch des Haares führt. Bei der Verwendung von Cysteamin kommt es deshalb immer wieder zu Reklamationen.

Da der Glycerinester und Cysteamin nur mit deutlichen Einschränkungen zur Haarumformung verwendbar sind, bleibt für die Praxis vor allem die Thioglykolsäure, gegebenenfalls auch in Kombination mit DL-Cystein bzw. L-Cystein und Thiomilchsäure.

Zwecks Verminderung der Haarschädigung bei Einsatz von Thioglykolsäure sind in den letzten Jahren Präparate auf den Markt gekommen, bei denen mit einem niedrigeren pH-Wert von etwa 7,5 gearbeitet wird. Allerdings müssen diese Präparate aus technischen Gründen mit zwei Teilen A und B angeboten und entsprechend verpackt werden. A und B werden erst kurz vor Gebrauch vermischt. Für dieses Verfahren ist, entsprechend den beiden Produktteilen, ein höherer Verpackungsaufwand erforderlich. In der Regel sind teure Spezialverpackungen im Einsatz.

Es ist weiterhin bekannt, daß bei der Haarumformung nach der Einwirkung der reduzierenden Lösung eine zusätzliche Keratinschädigung und Haarquellung durch den Spülvorgang mit Wasser erfolgt. Es geht deshalb in der Praxis auch darum, diese Schädigung soweit wie möglich zu verhindern.

Aus der EP-A-0 443 356 ist ein Verfahren zur dauerhaften Verformung von Keratinfasern bekannt, bei dem die einer reaktiven reduzierenden Lösung ausgesetzten Keratinfasern - zur Verhinderung einer schädlichen Haarquellung - nach der Einwirkungszeit mit einer wäßrigen Lösung von gegenüber den Fasern inerten Stoffen, insbesondere Alkali- oder Erdalkalisalzen, gespült werden.

Dort ist mit Beispiel 12 ein Haardauerverformungsverfahren beschrieben, bei dem man das Haar auf Wickler wickelt, 15 min lang eine alkalische Reduktionslösung einwirken läßt, ohne vorheriges Spülen mit Wasser, 10 min lang mit einer sauren wäßrigen Zwischenspülung vom von pH 5,9, enthaltend Betain, spült, die überschüssige Lösung abtupft und sodann die auf dem Wickler befindliche Strähne mit der Fixierlösung durchtränkt.

Den Verformungsverfahren des Standes der Technik ist jedoch gemeinsam, daß die reduzierenden Dauerverformungsmittel über die gesamte, für die Haarverformung erforderliche Zeit in unverminderter Stärke auf das Haar einwirken und dadurch, insbesondere bei mehrfacher Haarverformung, zu irreversiblen Haarschädigungen, fachlich als "Überkrausung" bezeichnet, führen. Die negativen Folgen für das Haar sind mangelnde Elastizität und Sprungkraft, verminderte Reißfestigkeit, schlechte Frisierbarkeit und verminderter Glanz.

Eine andere Möglichkeit zur Verbesserung der Pflegeeigenschaften besteht darin, die Reduktion der Haare vollständig bei niedrigerem pH-Wert durchzuführen (saure beziehungsweise Neutraldauerwelle). Dadurch werden Nebenreaktionen wie die Hydrolyse von Peptidbindungen, die zu erhöhter Haarschädigung führen, minimiert. In der Vergangenheit hat man daher versucht, diese Nachteile durch die Schaffung sogenannter "saurer Dauerwellmittel" zu überwinden, deren pH-Wert in der gebrauchsfertigen Form im Bereich von 6,8 bis 7,6 liegt. Als Reduktionsmittel wurde in diesem pH-Bereich Thioglykolsäureglycerinester verwendet. Wegen gelegentlich auftretender Sensibilisierungen will man jedoch auf den Einsatz dieser Verbindung verzichten.

Es besteht deshalb ein Bedarf nach einem Verfahren zur dauerhaften Verformung von Haaren, das ähnlich gute Ergebnisse hinsichtlich Qualität der Verformung, Elastizität und Sprungkraft der Locke und der Pflegeeigenschaften, wie Griff und Kämmbarkeit des Haares liefert, wie die sogenannte "saure Dauerwelle", wobei jedoch die Gefahr einer Sensibilisierung nicht besteht.

Es wurde nunmehr überraschend gefunden, daß dies für ein Verfahren nach Anspruch 1 zutrifft.

Bei dem erfindungsgemäßen Verfahren wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewikkelt. Sodann wird das Haar mit einer für die Haarverformung ausreichenden Menge, vorzugsweise 60 bis 120 Gramm, eines alkalischen Verformungsmittels auf der Basis einer haarkeratinreduzierenden Substanz behandelt.

Als haarkeratinreduzierende Substanz sind alle üblichen derartigen Stoffe geeignet. Es können insbesondere Thioglykolsäure, Thioglykolsäureamide, Thiomilchsäure, 3-Mercapto-propionsäure, Cystein, Cysteamin, Alkyl- oder Acylcysteamine oder die Salze dieser Verbindungen, auch im Gemisch miteinander, eingesetzt werden. Das Verformungsmittel enthält jedoch keinen Thioglykolsäureglycerinester.

Die in dem Dauerverformungsmittel enthaltenen haarkeratinreduzierenden Wirkstoffe werden in dem alkalischen Mittel zur dauerhaften Haarverformung bevorzugt in einer Menge von 2 bis 20 Gewichtsprozent, besonders bevorzugt in einer Menge von 4 bis 13 Gewichtsprozent und ganz besonders bevorzugt in einer Menge von 6 bis 10 Gewichtsprozent eingesetzt.

Neben den genannten oder anderen, an sich bekannten Reduktionsmitteln, enthalten sie üblicherweise ein Alkalisierungsmittel, dessen Konzentration von Art und Menge des haaarkeratinreduzierenden Wirkstoffs abhängt. Die Dauerverformungsmittel enthalten als Alkalisierungsmittel bzw. als Mittel zur Einstellung des pH-Wertes insbesondere Ammoniak, Natronlauge oder wasserlösliche, physiologisch verträgliche organische Basen, wie z.B. Di- oder Triethanolamin. Bevorzugtes Alkalisierungsmittel ist Ammoniak, wobei, je nach haaarkeratinreduzierendem Wirkstoff, die Einstellung des pH-Wertes auf den alkalischen Bereich zwischen 7,1 und 11, vorzugsweise 7,5 bis 9,5, angestrebt wird.

Das Verformungsmittel kann sowohl in Form einer wäßrigen Lösung oder einer Emulsion als auch in verdickter Form auf wäßriger Basis, insbesondere als Creme, Gel oder Paste vorliegen.

Selbstverständlich kann das Verformungsmittel alle für derartige Mittel üblichen und bekannten Zusatzstoffe, zum Beispiel Verdickungsmittel, wie beispielsweise, Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; Alkohole, wie zum Beispiel Ethanol, Propanol, Isopropanol und Glycerin; Zucker wie z. B. D-Glucose; Lösungsvermittler, Stabilisatoren, Puffersubstanzen, Parfümöle, Farbstoffe sowie haarkonditionierende und haarpflegende Bestandteile, wie zum Beispiel kationische Polymere, z. B. Polydimethyldiällylammoniumchlorid (CTFA Polyquaternium-6), Polydimethyl-aminoethylmethacrylat (zu 75% quaternisiert mit Diethylsulfat CTFA Polyquaternium-11), CTFA Polyquaternium-4, CTFA Polyquaternium-5, CTFA Polyquaternium-7, CTFA Polyquaternium-9, CTFA Polyquaternium-10, CTFA Polyquaternium-14, CTFA Polyquaternium-16, CTFA Polyquaternium-22, Lanolinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 1 bis 30 Gewichtsprozent sowie zur Vermeidung einer Überkrausung der Haare Dithioverbindungen, beispielsweise Dithiodiglykolsäure, Dithiomilchsäure, die Dithiole der genannten Verbindungen oder die jeweiligen Salze, zugesetzt werden.

Nach einer für die dauerhafte Verformung des Haares noch nicht ausreichenden Einwirkungszeit, welche je nach Haarbeschaffenheit, dem pH-Wert und der Verformungswirksamkeit des Verformungsmittels sowie in Abhängigkeit von der Anwendungstemperatur 2 bis 10 Minuten (7 bis 10 Minuten lang bei Raumtemperatur; 2 bis 6 Minuten lang bei erhöhter Temperatur) beträgt, werden die Wikkel, ohne vorheriges Spülen mit Wasser, mit 50 bis 2000 ml, vorzugsweise 300 ml bis 1000 ml und besonders bevorzugt 300 bis 600 ml, einer sauren wäßrigen Zwischenspülung vom pH = 2 bis 6,8, enthaltend eine aliphatische organische Säure, eine haarpflegende und haarkonditionierende Komponente und mindestens 50 Gew. % Wasser, gespült bzw. behandelt. Vorzugsweise enthält die saure Zwischenspülung 60 bis 96 Gew.%, besonders bevorzugt 80 bis 95 Gew.%, Wasser.

Unter erhöhter Temperatur wird eine Temperatur von 35 bis 60 Grad Celsius, vorzugsweise 40 bis 50 Grad Celsius, verstanden.

Als aliphatische organische Säure sind insbesondere die physiologisch verträglichen Säuren Zitronensäure, Weinsäure, Milchsäure, Essigsäure, Glyoxylsäure, Maleinsäure und Fumarsäure geeignet. Diese Säuren können allein oder im Gemisch miteinander, je nach Menge der für die Spülung vorgesehenen wäßrigen Zwischenspülung, in einer Menge von 0,05 bis 3 Gew.% enthalten sein, wobei der bevorzugt eingestellte pH-Wert 2,5 bis 4,5 beträgt.

Die haarpflegende und haarkonditionierende Komponente besteht vorzugsweise aus einer Zubereitung zur Pflege und Konditionierung des Haares, enthaltend
- 65 bis 98 Gew.% einer ersten, lipophilen Phase,
- 2 bis 35 Gew.% einer zweiten, amphiphilen Phase und
- 0 bis 5 Gew.% Wasser,
wobei die erste Phase aus wenigstens einer pflegenden bzw. konditionierend wirkenden lipophilen Wirksubstanz und die zweite Phase aus wenigstens einem amphiphilen Stoff besteht, der ausgewählt ist aus einer Stoffgruppe, die jeweils mit 2 - 200 Ethylenoxidgruppen oxethylierte und jeweils 6 - 60 C-Atome enthaltende Fettsäuren, Fettsäureester, Fettsäureamine und Fettsäureamide umfaßt. Solche geeigneten haarpflegenden und haarkonditionierenden Komponenten sind in der WO-A 97/09028 der Anmelderin beschrieben, worauf ausdrücklich Bezug genommen wird.

Die verwendete saure wäßrige Zwischenspülung kann als haarpflegende und haarkonditionierende Komponente das Gemisch aus 65 bis 98 Gew.% eines natürlichen oder synthetischen Öls und 2 bis 35 Gew.% eines polyoxethylierten, hydrierten Rizinusöls enthalten.

Besonders bevorzugt ist die haarpflegende und haarkonditionierende Komponente ein nachstehend mit "Ölkomplex" bezeichnetes Gemisch aus

### Ölkomplex

| | |
|---|---|
| Jojobaöl | 33,0 Gew.% |
| Sonnenblumenöl | 32,0 Gew.% |
| Avocadoöl | 31,0 Gew.% |
| mit 40 Ethylenoxidgruppen oxethyliertes Rizinusöl | 2,0 Gew.% |
| mit 7 Ethylenoxidgruppen oxethyliertes Rizinusöl | 1,0 Gew.% |
| Antioxidans | 0,1 Gew.% |
| Wasser | 0,9 Gew.% |
| | 100,0 Gew.% |

Die haarpflegende und haarkonditionierende Komponente kann auch ein kationisches Polymer, wie es beispielsweise vorstehend als Bestandteil des Dauerverformungsmittels genannt wurde, oder Lanolin, Lecithin, Glycerin, Allantoin, Purcellinöl, Silikonöl, Walrat, Wollwachs, Paraffinöl, Bienenwachs, Harnstoff, Betain, eine aliphatische von Mercaptogruppen freie Aminosäure, niedrigsiedende Isoparaffine, Eiweiß-Fettsäurekondensat, Keratinhydrolysat, Cholesterin oder Pantothensäure sein. Sie ist in der sauren wäßrigen Zwischenspülung allein oder im Gemisch in einer Menge von 0,1 bis 5 Gew% enthalten.

In einer weiteren bevorzugten Ausführungsform des erfindungsgemäßen Verfahrens werden die Wickel nach einer für die dauerhafte Verformung des Haares noch nicht ausreichenden Einwirkungszeit, ohne vorheriges Spülen mit Wasser, mit 50 bis 2000 ml, vorzugsweise 50 bis 600 ml und besonders bevorzugt 60 bis 100 ml, einer sauren wäßrigen Zwischenspülung vom pH = 2 bis 6,8, enthaltend eine aliphatische organische Säure, als haarpflegende und haarkonditionierende Komponente Betain und/oder eine aliphatische, von Mercaptogruppen freie Aminosäure sowie mindestens 50 Gew. % Wasser, behandelt.

Als aliphatische, von Mercaptogruppen freie Aminosäure wird vorzugsweise Glycin, Alanin, Methionin, Valin, Leucin, Isoleucin, Glutamin, Serin, Tyrosin, Threonin, Asparagin, jeweils allein oder im Gemisch, in einer Konzentration von 0,5 bis 10 Gew.% verwendet.

Zusätzlich können in der verwendeten sauren wäßrigen Zwischenspülung auch vorteilhaft Salze mehrwertiger Metalle, insbesondere des Magnesiums, Calciums oder des Aluminiums, z. B. MgCl₂, AlCl₃ und MgSO₄, in einer Menge von 0,5 bis 5 Gew.% enthalten sein.

Die saure wäßrigen Zwischenspülung wird auf das Haar wird bei der selben Temperatur mindestens doppelt so lang einwirken gelassen, wie die Einwirkungsdauer des alkalischen Haarverformungsmittel betragen hat. Die Einwirkungsdauer der sauren wäßrigen Zwischenspülung beträgt daher, je nach Anwendungstemperatur und Einwirkungsdauer des alkalischen Haarverformungsmittels, 8 bis 30 Minuten, vorzugsweise 10 bis 20 Minuten.

Wenn die alkalische Reduktionsstufe bei Raumtemperatur durchgeführt wird, ist es auch möglich, die saure Zwischenspülung bei erhöhter Temperatur für die gleiche Zeit wie das alkalische Dauerverformungsmittel auf das Haar einwirken zu lassen.

Unter erhöhter Temperatur wird eine Temperatur von 35 bis 60 Grad Celsius, vorzugsweise 40 bis 50 Grad Celsius, verstanden.

Vorzugsweise führt man die alkalische Reduktionsstufe und die saure Reduktionsstufe des erfindungsgemäßen Verfahrens jeweils bei einer erhöhten Temperatur von 40 bis 50 Grad Celsius aus, wobei dann die Einwirkungszeiten während der alkalischen Reduktionsstufe 6 bis 8 Minuten und während der sauren Reduktionsstufe 12 bis 20 Minuten betragen.

Gegebenenfalls wird nun überschüssige Flüssigkeit von den Wickeln abtupft.

Nach dieser sauren Zwischenspülung kann das Haar gegebenenfalls mit Wasser gespült werden. Danach ist es vorteilhaft, das Haar mit einem Handtuch abzutupfen um es für die Aufnahme des Fixiermittels vorzubereiten.

Sodann wird das Haar oxidativ nachbehandelt ("fixiert"). Das Fixiermittel wird, je nach Haarfülle, vorzugsweise in einer Menge von 80 bis 100 Gramm, angewendet. Für die oxidative Nachbehandlung im aufgewickelten oder abgewickelten Zustand kann jedes beliebige, für eine derartige Behandlung geeignete Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationische Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.% in dem Nachbehandlungsmittel enthalten sein.

Anschließend werden die Wickler entfernt. Falls erforderlich, kann das abgewikkelte Haar nun nochmals oxidativ nachbehandelt werden. Sodann wird das Haar mit Wasser gespült, gegebenenfalls zur Wasserwelle gelegt und schließlich getrocknet.

Es hat sich gezeigt, daß durch Anwendung des erfindungsgemäßen Verfahrens sich ein gutes Ergebnis hinsichtlich der Kämmbarkeit und des Griffes des Haares erzielen läßt. Zusätzlich wurde überraschender Weise festgestellt, daß die Haltbarkeit, Elastizität und Sprungkraft der Locke höher ist, als bei herkömmlich verformten Haaren.

Die nachfolgenden Ausführungsbeispiele dienen der näheren Illustration der Erfindung

### Beispiel 1

Durch Farbbehandlung vorgeschädigtes, 15 cm langes Haar wird mit einem Shampoo gewaschen, frottiert und auf Wickler mit einem Durchmesser von 6 bis 8 mm gewickelt. Anschließend wird das alkalische Dauerwellmittel vom pH = 8,0 der nachfolgenden Zusammensetzung gleichmäßig auf dem gewickelten Haar verteilt.

| | |
|---|---|
| 15,2 Gew.% | Ammoniumthioglykolat, 70%ig |
| 3,5 Gew.% | Ammoniumhydrogencarbonat |
| 1,0 Gew.% | Ammoniak, 25%ig |
| 0,8 Gew.% | Polydimethyldiallylammoniumchlorid |
| 1,5 Gew.% | Harnstoff |
| 78,0 Gew.% | Wasser, vollentsalzt |
| 100,0 Gew.% | |

Nach einer Einwirkungszeit von 8 Minuten bei einer, durch Anwendung eines Infrarot-Strahlungsgerätes erhöhten Temperatur von 40°C, werden die Wickel auf der linken Kopfseite mit 250 ml einer sauren wäßrigen Zwischenspülung vom pH = 2,6 der Zusammensetzung

| | |
|---|---|
| 0,4 Gew.% | Milchsäure |
| 2,8 Gew.% | Magnesiumsulfat |
| 4,0 Gew.% | Ölkomplex, wie vorstehend beschrieben |
| 92,8 Gew.% | Wasser |
| 100,0 Gew.% | |

gespült, während die Wickel der rechten Kopfseite mit 250 ml Wasser gespült werden.

Nach einer Einwirkungszeit von 16 Minuten bei einer, durch Anwendung eines Infrarot-Strahlungsgerätes erhöhten Temperatur von 40°C, werden die Wickel beider Kopfseiten jeweils mit viel Wasser gespült und sodann mit einer Serviette abgetupft. Anschließend wird das Haar mit 70 ml einer 2,5 %igen wäßrigen Wasserstoffperoxidlösung oxidativ nachbehandelt. Nach Entfernung der Wickler werden die Haare erneut mit Wasser gespült und anschließend getrocknet. Als Ergebnis dieser Behandlung zeigte das Haar auf der linken, mit der sauren wäßrigen Zwischenspülung behandelten Kopfseite gegenüber der rechten Kopfseite eine deutlich höhere Elastizität und Sprungkraft der Locke sowie besseren Griff und Naßkämmbarkeit.

### Beispiel 2

Normales, nicht vorgeschädigtes 15 cm langes Haar wird mit einem Shampoo gewaschen, frottiert und auf Wickler mit einem Durchmesser von 6 bis 8 mm gewickelt. Anschließend wird das alkalische Dauerwellmittel vom pH = 8,6 der nachfolgenden Zusammensetzung gleichmäßig auf dem gewickelten Haar verteilt.

| | |
|---|---|
| 17,0 Gew.% | Ammoniumthioglykolat, 70%-ig |
| 3,4 Gew.% | Ammoniumthiolactat, 70%-ig |
| 1,8 Gew.% | Ammoniak, 25%-ige wäßrige Lösung |
| 0,5 Gew.% | Polydimethyldiallylammoniumchlorid |
| 3,0 Gew.% | Harnstoff |
| 1,5 Gew.% | 1,2-Propylenglykol |
| 72.8 Gew.% | Wasser |
| 100,0 Gew.% | |

Nach einer Einwirkungszeit von 6 Minuten bei einer, durch Anwendung eines Infrarot-Strahlungsgerätes erhöhten Temperatur von 40°C, werden die Wickel auf der linken Kopfseite mit 250 ml einer sauren wäßrigen Zwischenspülung vom pH = 2,8 der Zusammensetzung

| | |
|---|---|
| 0,5 Gew.% | Zitronensäure |
| 0,5 Gew.% | Keratinhydrolysat, quaternisiert |
| 0,6 Gew.% | Cetyltrimethylammoniumchlorid |
| 1,0 Gew.% | Dimethylcarboxymethyl-kokosfettsäureamidoammoniumbetain (Rewoteric® AM B 14) |
| 97,4 Gew.% | Wasser |
| 100,0 Gew.% | |

gespült, während die Wickel der rechten Kopfseite mit 250 ml Wasser gespült werden.

Nach einer Einwirkungszeit von 20 Minuten werden die Haare wie in Beispiel 1 weiterbehandelt.

Als Ergebnis dieser Behandlung zeigte das Haar auf der linken, mit der sauren wäßrigen Zwischenspülung behandelten Kopfseite gegenüber der rechten Kopfseite eine deutlich höhere Elastizität und Sprungkraft der Locke sowie besseren Griff und Naßkämmbarkeit.

### Beispiel 3

30 cm lange gefärbte Haare werden mit einem üblichen Shampoo gewaschen, leicht mit einem Handtuch angetrocknet und dann auf Wickler von 6 bis 8 mm Durchmesser aufgerollt. Anschließend werden über den ganzen Kopf etwa 80 ml der nachfolgenden Wellösung auf die Wickel aufgetragen.

### Wellösung:

| | |
|---|---|
| 10,90 Gew.% | Thioglykolsäure rein |
| 9,10 Gew.% | Ammoniak 25%ig |
| 5,50 Gew.% | Ammoniumhydrogencarbonat |
| 0,40 Gew.% | mit 45 Mol EO ethoxylierters Rizinusöl |
| 0,05 Gew.% | Parfüm |
| 1,00 Gew.% | Polydimethyldiallylammoniumchlorid, 40 %ige wäßrige |
| | Lösung |
| 1,00 Gew.% | Vinylpyrrolidon/Dimethylaminoethylmethacrylat-Copolymer, 19 %ige wäßrige Lösung |
| 72,05 Gew.% | Wasser |
| 100,00 Gew.% | pH-Wert: 8,1 |

Die Wellösung wird auf der linken Kopfhälfte 6 Minuten lang bei Raumtemperatur und auf der rechten Seite 12 Minuten lang bei Raumtemperatur einwirken gelassen. Während der Einwirkungszeit werden 20 ml der nachfolgenden Zwischenspülung mit 40 ml Wasser gemischt und in zwei Teile A und B zu je 30 ml aufgeteilt.

### Zwischenspülung als Emulsion:

| | |
|---|---|
| 0,70 Gew.% | Cetylstearylalkohol (50:50) |
| 0,60 Gew.% | Laurylalkoholpolyglykolether (2 EO) |
| 0,70 Gew.% | Vaseline |
| 0,30 Gew.% | PHB-Methylester |
| 0,30 Gew.% | Natrium-Cetylstearylsulfat |
| 10,00 Gew.% | Betain |
| 1,00 Gew.% | Zitronensäure Anhydro |
| 86,40 Gew.% | Wasser |
| 100,00 Gew.% | pH-Wert: 2,5 |

Auf der linken Seite werden 30 ml der verdünnten Zwischenspülung (Teil A) nach 6 Minuten Einwirkungszeit aufgetragen, danach weitere 20 Minuten gewartet und dann mit warmem Wasser ausgespült.

Auf der rechten Seite werden nach Ablauf von 12 Minuten 30 ml der verdünnten Zwischenspülung (Teil B) aufgetragen, 10 Minuten gewartet und dann mit warmem Wasser ausgespült.

Nach dem Spülen werden werden die Wickel auf beiden Seiten mit einem Handtuch abgetupft und jeweils mit der nachfolgenden Fixierung fixiert.

### Fixierung:

| | |
|---|---|
| 0,25 Gew.% | o-Phosphorsäure 85%ig |
| 4,83 Gew.% | Wasserstoffperoxid 50%ig |
| 0,40 Gew.% | mit 45 Mol EO ethoxylierters Rizinusöl |
| 0,05 Gew.% | Parfüm |
| 1,00 Gew.% | Polydimethyldallylammoniumchlorid, 40 %ige wäßrige |
| | Lösung |
| 1,00 Gew.% | Vinylpyrrolidon/Dimethylaminoethylmethacrylat-Copolymer, 19 %ige wäßrige Lösung |
| 92,47 Gew.% | Wasser |
| 100,00 Gew.% | |

Nach der vorstehenden Versuchsanordnung wurden 6 Modelle jeweils im Abstand von 8 Wochen dreimal behandelt. Nach der dritten Behandlung waren folgende Unterschiede - links im Vergleich zu rechts - festzustellen:
- links:: bessere Elastizität der Welle (nasser Zustand des Haares nach der Fixierung)
- links:: bessere Nasskämmbarkeit direkt nach Fixierung
- links:: Haargriff trocken besser, weniger stumpf
- links:: gleichmäßigeres Wellbild und besserer Haarzustand

Die Versuche zeigen eindeutig, daß das Haar mit der erfindungsgemäßen Methode weniger geschädigt wird bei praktisch gleicher Wellintensität. Dies ist ein wesentlicher und überraschender Fortschritt und entspricht der Zielsetzung der vorliegenden Erfindung.

### Beispiel 4

Hierfür werden die gleiche Präparate wie für Beispiel 3 verwendet, aber die Versuchsanordnung etwas modifiziert. Sowohl auf der linken, als auf der rechten Kopfhälfte wird mit je 6 Minuten Einwirkungszeit der Wellösung gearbeitet. Danach werden auf der linken Kopfhälfte 30 ml der verdünnten Zwischenspülung (Teil A) auf die gewickelten Haare aufgetragen, mit einem Handtuch leicht abgetupft und 20 Minuten gewartet. Danach wird mit warmem Wasser gespült und mit der Fixierung nach Beispiel 3 fixiert.

Im Gegensatz zur linken Kopfseite wird auf der rechten Kopfseite nach 6 Minuten Einwirkungszeit sofort mit warmem Wasser gespült, ohne daß Teil B der verdünnten Zwischenspülung zur Anwendung kommt. Nach dem Spülen mit Wasser wird sofort mit der Fixierung nach Beispiel 3 fixiert.

Das Ergebnis von Beispiel 4 ist, daß die Haare der linken Kopfhälfte, sowohl am Ansatz als auch in den Längen und Spitzen deutlich stärker umgeformt sind, vergleichsweise zur rechten Kopfhälfte.

Mit Beispiel 4 wird gezeigt, daß die Haarwellung auch dann noch deutlich fortschreitet, wenn die Zwischenspülung angewandt und der pH-Wert des einwirkenden Verformungsmittel/Zwischenspülung-Gemisches abgesenkt ist.

### Beispiel 5

30 cm lange poröse oder gefärbte Haare werden mit einem normalen Shampoo gewaschen, leicht mit einem Handtuch angetrocknet und aufgewickelt. Sodann werden 80 ml Wellösung nach Beispiel 3 auf die Wickel aufgetragen und bei Raumtemperatur 6 Minuten lang einwirken gelassen. Danach wird auf der linken Seite mit 500 ml warmem Wasser ausgespült, während gleichzeitig auf der rechten Seite mit einer Mischung, bestehend aus 400 ml Wasser und 100 ml der Zwischenspülung nach Beispiel 3, gespült wird.

Nach dem Spülvorgang werden die Haare leicht mit einem Handtuch angetrocknet und es wird 20 Minuten lang gewartet. Danach wird das Haar auf dem ganzen Kopf in üblicher Weise mit der Fixierlösung nach Beispiel 3 fixiert.

Der Verfahrensablauf für links und rechts ist annähernd gleich, aber mit dem Unterschied, daß die Beschaffenheit des Spülmittels unterschiedlich ist. Deshalb werden folgende Unterschiede nach Einwirkung des Fixiermittels bezogen auf das nasse Haar gefunden:
- links:: deutlich stumpferer Griff des Haares
- links:: deutlich schlechtere Nasskämmbarkeit
- links:: Gleichmäßigkeit und Elastizität der Welle schlechter

Diese Ergebnisse belegen eindeutig, daß mit der erfindungsgemäßen Methode die Haare weniger geschädigt werden. Hinzu kommt der merkbar stumpfere und rauhe Haargriff auf der linken Haarseite nach dem Trocknen der Haare.

Mit den vorliegenden Beispielen wird weiterhin gezeigt, daß die Vorteile des neuen Verfahrens praxisgerecht umgesetzt werden können, wobei die Möglichkeit besteht, nach Anwendung der Zwischenspülung zusätzlich mit Wasser auszuspülen oder auf den Spülvorgang mit Wasser zu verzichten.

### Beispiel 6

An Stelle einere emulsionsförmigen Zwischenspülung nach Beispiel 3 kann auch eine Zwischenspülung in Form einer Lösung der folgenden Zusammensetzung verwendet werden:

### Zwischenspülung:

| | |
|---|---|
| 5,00 Gew.% | Glycin |
| 1,00 Gew.% | Zitronensäure Anhydro |
| 2,00 Gew.% | Dimethyldiallylammoniumchlorid, 40 %ige wäßrige Lösung |
| 2,00 Gew.% | Kokosalkyldimethylammoniumbetain, 30 %ige wäßrige Lösung |
| 2,00 Gew.% | PEG-7-Glycerylcocoate |
| 88,00 Gew.% | Wasser |
| 100,0 Gew.% | |

Bei Anwendung der Zwischenspülung nach Beispiel 6 an Stelle der Zwischenspülung nach Beispiel 3 wurden vergleichbare Resultate erhalten.

## Patentansprüche

1. Verfahren zur dauerhaften Verformung von Haaren, bei dem man
a) das Haar auf Wickler aufwickelt,
b) auf das gewickelte Haar ein alkalisches Dauerverformungsmittel auf der Basis einer haarkeratinreduzierenden Substanz aufbringt,
c) das Dauerverformungsmittel auf das Haar einwirken läßt,
d) die Wickel, ohne vorheriges Spülen mit Wasser mit 50 bis 2.000 ml einer sauren wäßrigen Zwischenspülung vom von pH 2 bis 6,8, enthaltend eine haarpflegende und haarkonditionierende Komponente und mindestens 50 Gew. % Wasser, behandelt,
e) gegebenenfalls überschüssige Flüssigkeit von den Wickeln abtupft,
f) die Zwischenspülung einwirken läßt,
g) gegebenenfalls mit Wasser spült,
h) das Haar vor und/oder nach dem Abwickeln mit einem Fixiermittel auf der Basis eines Oxidationsmittels behandelt und
i) nach der Einwirkungszeit das Fixiermittel mit Wasser aus dem Haar ausspült oder mit einem Shampoo auswäscht,
**dadurch gekennzeichnet, daß**
(I) man das Dauerverformungsmittel in der Stufe c) 7 bis 10 Minuten lang bei Raumtemperatur oder 2 bis 6 Minuten lang bei erhöhter Temperatur auf das Haar einwirken läßt,
(II) die saure wäßrige Zwischenspülung der Stufe d) eine aliphatische organische Säure enthält und
(III) man die Zwischenspülung in der Stufe f) 10 bis 30 Minuten lang bei Raumtemperatur oder 5 bis 15 Minuten lang bei erhöhter Temperatur einwirken läßt, wobei die Einwirkungsdauer gemäß Verfahrensschritt f) bei der selben Temperatur mindestens das Doppelte der Einwirkungsdauer von Verfahrensschritt c) ist.

2. Verfahren nach Anspruch 1, dadurch gekennzeichnet, daß die erhöhte Temperatur 40 bis 50 Grad Celsius beträgt.

3. Verfahren nach einem der Ansprüche 1 oder 2, dadurch gekennzeichnet, daß man das Dauerverformungsmittel bei einer Temperatur von 40 bis 50 Grad Celsius 6 bis 8 Minuten lang einwirken läßt.

4. Verfahren nach einem der Ansprüche 1 bis 3, dadurch gekennzeichnet, daß man ein Dauerverformungsmittel verwendet, indem die haarkeratinreduzierende Substanz Thioglykolsäure oder dessen Salz ist.

5. Verfahren nach einem der Ansprüche 1 bis 4, dadurch gekennzeichnet, daß man die wäßrige Zwischenspülung bei einer Temperatur von 40 bis 50 Grad Celsius 12 bis 20 Minuten lang einwirken läßt.

6. Verfahren nach einem der Ansprüche 1 bis 5, dadurch gekennzeichnet, daß man die saure wäßrige Zwischenspülung in einer Menge von 300 bis 600 ml anwendet.

7. Verfahren nach einem der Ansprüche 1 bis 6, dadurch gekennzeichnet, daß das man in der wäßrigen Zwischenspülung als aliphatische Säure Milchsäure oder Zitronensäure verwendet.

8. Verfahren nach einem der Ansprüche 1 bis 7, dadurch gekennzeichnet, daß die saure wäßrige Zwischenspülung als haarpflegende und haarkonditionierende Komponente mindestens eine Subsanz, ausgewählt aus Betain und einer aliphatischen, von Mercaptogruppen freien Aminosäure enthält.

9. Verfahren nach Anspruch 8, dadurch gekennzeichnet, daß man in der sauren wäßrigen Zwischenspülung eine von Mercaptogruppen freie Aminosäure, ausgewählt aus Glycin, Alanin, Methionin, Histidin, Valin, Leucin, Isoleucin, Tryptophan, Kreatinin, Lysin, Glutamin, Prolin, Serin, Tyrosin, Threonin und Asparagin, verwendet.

10. Verfahren nach einem der Ansprüche 8 oder 9, dadurch gekennzeichnet, daß man die saure wäßrige Zwischenspülung in einer Menge von 50 bis 600 ml anwendet.

11. Verfahren nach einem der Ansprüche 1 bis 7, dadurch gekennzeichnet, daß die saure wäßrige Zwischenspülung als haarpflegende und haarkonditionierende Komponente eine Zubereitung zur Pflege und Konditionierung des Haares, enthaltend
- 65 bis 98 Gew.% einer ersten, lipophilen Phase,
- 2 bis 33 Gew.% einer zweiten, amphiphilen Phase und
- 0 bis 5 Gew.% Wasser,
wobei die erste Phase aus wenigstens einer pflegenden bzw. konditionierend wirkenden lipophilen Wirksubstanz und die zweite Phase aus wenigstens einem amphiphilen Stoff besteht, der ausgewählt ist aus einer Stoffgruppe, die jeweils mit 2 bis 200 Ethylenoxidgruppen oxethylierte und jeweils 6 bis 60 C-Atome enthaltende Fettsäuren, Fettsäureester, Fettsäureamine und Fettsäureamide umfaßt, enthält.

12. Verfahren nach Anspruch 11, dadurch gekennzeichnet, daß die verwendete saure wäßrige Zwischenspülung als haarpflegende und haarkonditionierende Komponente 65 bis 98 Gew% eines natürlichen oder synthetischen Öls und 2 bis 35 Gew.% eines polyoxethylierten, hydrierten Rizinusöl enthält.

13. Verfahren nach nach Anspruch 12, dadurch gekennzeichnet, daß die saure wäßrige Zwischenspülung als haarpflegende und haarkonditionierende Komponente folgendes enthält:
| | |
|---|---|
| Jojobaöl | 33,0 Gew.% |
| Sonnenblumenöl | 32,0 Gew.% |
| Avocadoöl | 31,0 Gew.% |
| mit 40 Ethylenoxidgruppen oxethyliertes Rizinusöl | 2,0 Gew.% |
| mit 7 Ethylenoxidgruppen oxethyliertes Rizinusöl | 1,0 Gew.% |
| Antioxidans | 0,1 Gew.% |
| Wasser | 0,9 Gew.%. |

## Claims

1. Process for the permanent shaping of hair, in which
a) the hair is wound onto curlers,
b) an alkaline permanent shaping agent based on a hair-keratin-reducing substance is applied to the wound hair,
c) the permanent shaping agent is allowed to take effect on the hair,
d) the curlers, without prior rinsing with water, are treated with from 50 to 2000 ml of an acidic aqueous intermediate rinse having a pH of from 2 to 6.8 and containing a hair-care and hair-conditioning component and at least 50% by weight of water,
e) excess fluid is optionally dabbed off the curlers,
f) the intermediate rinse is allowed to take effect,
g) rinsing is optionally carried out with water,
h) the hair, before and/or after being unwound, is treated with a setting agent based on an oxidising agent and
i) after the period of action, the setting agent is rinsed out of the hair with water or washed out with a shampoo,
**characterised in that**
(I) the permanent shaping agent in stage c) is allowed to take effect on the hair for from 7 to 10 minutes at room temperature or from 2 to 6 minutes at elevated temperature,
(II) the acidic aqueous intermediate rinse of stage d) contains an aliphatic organic acid and
(III) the intermediate rinse in stage f) is allowed to take effect for from 10 to 30 minutes at room temperature or from 5 to 15 minutes at elevated temperature, the period of action according to process step f), at the same temperature, being at least double the period of action of process step c).

2. Process according to claim 1, characterised in that the elevated temperature is from 40 to 50 degrees Celsius.

3. Process according to either claim 1 or claim 2, characterised in that the permanent shaping agent is allowed to take effect at a temperature of from 40 to 50 degrees Celsius for from 6 to 8 minutes.

4. Process according to any one of claims 1 to 3, characterised in that a permanent shaping agent is used in which the hair-keratin-reducing substance is thioglycolic acid or its salt.

5. Process according to any one of claims 1 to 4, characterised in that the aqueous intermediate rinse is allowed to take effect at a temperature of from 40 to 50 degrees Celsius for from 12 to 20 minutes.

6. Process according to any one of claims 1 to 5, characterised in that the acidic aqueous intermediate rinse is applied in an amount of from 300 to 600 ml.

7. Process according to any one of claims 1 to 6, characterised in that lactic acid or citric acid is used as the aliphatic acid in the aqueous intermediate rinse.

8. Process according to any one of claims 1 to 7, characterised in that the acidic aqueous intermediate rinse contains as the hair-care and hair-conditioning component at least one substance selected from betaine and an aliphatic amino acid free from mercapto groups.

9. Process according to claim 8, characterised in that an amino acid which is free from mercapto groups and which is selected from glycine, alanine, methionine, histidine, valine, leucine, isoleucine, tryptophan, creatinine, lysine, glutamine, proline, serine, tyrosine, threonine and asparagine is used in the acidic aqueous intermediate rinse.

10. Process according to either claim 8 or claim 9, characterised in that the acidic aqueous intermediate rinse is applied in an amount of from 50 to 600 ml.

11. Process according to any one of claims 1 to 7, characterised in that the acidic aqueous intermediate rinse contains, as the hair-care and hair-conditioning component, a preparation for the care and conditioning of the hair, containing
- from 65 to 98% by weight of a first, lipophilic, phase,
- from 2 to 33% by weight of a second, amphiphilic, phase and
- from 0 to 5% by weight of water,
wherein the first phase comprises at least one lipophilic active substance having a care or conditioning action and the second phase comprises at least one amphiphilic substance selected from a substance group comprising fatty acids, fatty acid esters, fatty acid amines and fatty acid amides, each of which is oxyethylated with from 2 to 200 ethylene oxide groups and each of which contains from 6 to 60 carbon atoms.

12. Process according to claim 11, characterised in that the acidic aqueous intermediate rinse used contains, as the hair-care and hair-conditioning component, from 65 to 98% by weight of a natural or synthetic oil and from 2 to 35% by weight of a polyoxyethylated hydrogenated castor oil.

13. Process according to claim 12, characterised in that the acidic aqueous intermediate rinse contains the following as the hair-care and hair-conditioning component :
| | |
|---|---|
| jojoba oil | 33.0% by weight |
| sunflower oil | 32.0% by weight |
| avocado oil | 31.0% by weight |
| castor oil oxyethylated with 40 ethylene oxide groups | 2.0% by weight |
| castor oil oxyethylated with 7 ethylene oxide groups | 1.0% by weight |
| antioxidant | 0.1% by weight |
| water | 0.9% by weight. |

## Revendications

1. Procédé de déformation permanente des cheveux, dans lequel
a) on enroule les cheveux sur des rouleaux,
b) on applique sur les cheveux enroulés un produit alcalin de déformation permanente à base d'une substance qui réduit la kératine du cheveu,
c) on laisse agir le produit de déformation permanente sur les cheveux,
d) on traite les boucles, sans rinçage préalable à l'eau, avec 50 à 2000 ml d'un rinçage intermédiaire acide et aqueux d'un pH compris entre 2 et 6,8, contenant un composant de soins et de conditionnement des cheveux, ainsi qu'au moins 50 % en poids d'eau,
e) on enlève par tamponnement le liquide éventuellement excédentaire des boucles,
f) on laisse agir le rinçage intermédiaire,
g) on rince éventuellement avec de l'eau,
h) on traite les cheveux avant et/ou après enlèvement des rouleaux au moyen d'un fixatif à base d'un oxydant et
i) après le temps d'action, on enlève le fixatif des cheveux par rinçage à l'eau, ou par lavage au moyen d'un shampooing,
caractérisé en ce que
(I) on laisse agir le produit de déformation permanente sur les cheveux au cours de l'étape c) de 7 à 10 minutes à la température ambiante ou de 2 à 6 minutes à une température plus élevée,
(II) le rinçage intermédiaire acide et aqueux de l'étape d) contient un acide aliphatique organique et
(III) on laisse agir le rinçage intermédiaire au cours de l'étape f) de 10 à 30 minutes à la température ambiante ou de 5 à 15 minutes à une température supérieure, la durée d'action selon l'étape f) du procédé à la même température étant au moins le double de la durée d'action de l'étape c) du procédé.

2. Procédé selon la revendication 1, caractérisé en ce que la température supérieure est de 40 à 50 degrés Celsius.

3. Procédé selon l'une des revendications 1 ou 2, caractérisé en ce qu'on laisse agir le produit de déformation permanente à une température de 40 à 50 degrés Celsius pendant six à huit minutes.

4. Procédé selon l'une des revendications 1 à 3, caractérisé en ce qu'on utilise un produit de déformation permanente dans lequel la substance qui réduit la kératine du cheveu est de l'acide thioglycolique ou son sel.

5. Procédé selon l'une des revendications 1 à 4, caractérisé en ce qu'on laisse agir le rinçage intermédiaire aqueux à une température de 40 à 50 degrés Celsius de 12 à 20 minutes.

6. Procédé selon l'une des revendications 1 à 5, caractérisé en ce qu'on utilise une quantité de 300 à 600 ml de rinçage intermédiaire acide et aqueux.

7. Procédé selon l'une des revendications 1 à 6, caractérisé en ce que dans le rinçage intermédiaire aqueux on utilise de l'acide lactique ou de l'acide citrique comme acide aliphatique.

8. Procédé selon l'une des revendications 1 à 7, caractérisé en ce que le rinçage intermédiaire acide et aqueux contient comme composants de soins et de conditionnement des cheveux, au moins une substance sélectionnée dans la bétaïne et un aminoacide aliphatique, exempt de groupes mercapto.

9. Procédé selon la revendication 8, caractérisé en ce qu'on utilise dans le rinçage intermédiaire acide et aqueux un aminoacide, exempt de groupes mercapto, sélectionné parmi la glycine, l'alanine, la méthionine, l'histidine, la valine, la leucine, l'isoleucine, le tryptophane, la créatinine, la lysine, la glutamine, la proline, la sérine, la tyrosine, la thréonine et l'asparagine.

10. Procédé selon l'une des revendications 8 ou 9, caractérisé en ce qu'on utilise une quantité de 50 à 600 ml de rinçage intermédiaire acide et aqueux.

11. Procédé selon l'une des revendications 1 à 7, caractérisé en ce que le rinçage intermédiaire acide et aqueux contient, en tant que composants de soins et de conditionnement des cheveux, une préparation pour les soins et le conditionnement des cheveux, qui contient
- de 65 à 98 % en poids d'une première phase lipophile,
- de 2 à 33 % en poids d'une deuxième phase amphiphile, et
- de 0 à 5 % en poids d'eau, la première phase étant constituée d'au moins une substance active lipophile à action soignante ou conditionnante, et la deuxième phase constituée d'au moins une substance amphiphile qui est choisie parmi un groupe de substances qui comprend des acides gras, des esters d'acides gras, des amines d'acides gras et des amides d'acides gras oxéthylés chacun avec 2 à 200 groupes d'éthylène oxyde et contenant chacun de 6 à 60 atomes de C.

12. Procédé selon la revendication 11, caractérisé en ce que le rinçage intermédiaire acide et aqueux utilisé contient comme composants de soins et de conditionnement des cheveux de 65 à 98 % en poids d'une huile naturelle ou synthétique et de 2 à 35 % en poids d'une huile de ricin polyoxéthylée et hydrogénée.

13. Procédé selon la revendication 12, caractérisé en ce que le rinçage intermédiaire acide et aqueux contient comme composants de soins et de conditionnement des cheveux, les composants suivants :
| | |
|---|---|
| Huile de jojoba | 33,0 % en poids |
| Huile de tournesol | 32,0 % en poids |
| Huile d'avocat | 31,0 % en poids |
| Huile de ricin oxéthylée avec 40 groupes d'éthylène oxyde | 2,0 % en poids |
| Huile de ricin oxéthylée avec 7 groupes d'éthylène oxyde | 1,0 % en poids |
| Antioxydant0,1 % en poids | |
| Eau | 0,9 % en poids |
